(19) Europäisches Patentamt
European Patent Office
Office européen des brevets

(11) Publication number: 0 292 682
A2

(12) # EUROPEAN PATENT APPLICATION

(21) Application number: 88105698.0

(51) Int. Cl.4: C07C 85/11 , C07C 87/60

(22) Date of filing: 11.04.88

(30) Priority: 28.05.87 IT 2069687

(43) Date of publication of application:
30.11.88 Bulletin 88/48

(84) Designated Contracting States:
BE DE FR GB NL

(71) Applicant: RIMAR CHIMICA S.p.A.
Località Colombara, 91
I-36070 Trissino (VI)(IT)

(72) Inventor: Carozza, Primo
c/o RIMAR CHIMICA S.p.A. Località
Colombara, 91
I-36070 Trissino VI(IT)
Inventor: Gandolfi, Vittorio
c/o RIMAR CHIMICA S.p.A. Località
Colombara, 91
I-36070 Trissino VI(IT)

(74) Representative: Trupiano, Roberto
BREVETTI EUROPA S.r.l. Piazza Bernini, 6
I-20133 Milano (MI)(IT)

(54) **Process for the catalytic reduction of aromatic nitro-halo-derivatives.**

(57) Process for reducing aromatic nitro-halo-derivatives by means of catalytic hydrogenation, in the presence of catalysts of supported Pd, and of a dehalogenation inhibitor system constituted by at least an organic ester of phosphorous acid and at least a hydrocarbyl-silane, in synergistic mixture, at a temperature of the order of from 50° to 100° C, under $H_2$ pressure. High conversions (higher than 99%) associated with low dehalogenation values (lower than 1%) are obtained.

EP 0 292 682 A2

Xerox Copy Centre

# PROCESS FOR THE CATALYTIC REDUCTION OF AROMATIC NITRO-HALO-DERIVATIVES

The present invention relates to a process for the catalytic reduction of aromatic nitro-halo-derivatives.

In particular, the present invention is concerned with a process for reducing aromatic nitro-halo-derivatives having the formula (I):

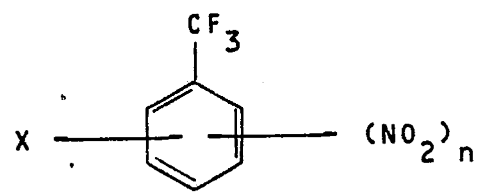

(I)

wherein X represents a halogen atom, preferably a chlorine atom, and n represents an integer selected from 1 and 2, in the presence of a catalytic system comprising palladium and dehalogenation inhibitor agents.

More particularly, the present invention relates to the reduction of the nitro-chloro-derivatives of benzotrifluoride having the formula (I) (with n = 1 and X = Cl). The corresponding aromatic, either mono- or difunctional halo-amines are obtained, which are important intermediate compounds for preparing industrial products highly interesting in the field of fine chemistry products (dyes, drugs, parasiticides, and so forth).

A particular product, which the process of the present invention is directed to, is constituted by 4-chloro-3-amino-benzotrifluoride, obtained from the corresponding 3-nitro-derivative by reduction.

It is known that the nitro-substituted aromatic products can be reduced to yield the corresponding amines by operating in the presence of metal catalysts, in particular noble metals. It is known, in particular, that the reduction of the aromatic nitrocompounds substituted with halogens, in particular if activated, poses particular problems due to the fact that the halogen bonded to the aromatic ring is easily subject to hydrogenolysis, and therefore during the process an undesired considerable dehalogenation occurs in the end product. Therefore, various catalytic systems were proposed, also associated with agents having a dehalogenation inhibiting activity, such as, e.g.,supported catalysts of rhodium, platinum, palladium, Raney nickel, and so forth, associated, or not, with inhibitor agents, such as oxides of calcium, magnesium, piperazine, morpholine and their N-alkyl-substituted derivatives, dicyanamide, (cyclo)-alkylamines, arylphosphites and silanes alone.

Nontheless, the above said technologies do not result always satisfactory. If the most widely used catalyst, i.e., Pt, and in particular Pt/C, is taken into consideration, on one hand good yields with an acceptable dehalogenation rate are obtained, but, on the other hand, the catalyst is very expensive, and furthermore is subject to a rapid poisoning, with the relevant operative and economic disadvantages. The use of inhibitor agents does not seem to improve this point. In case of other catalytic systems, with or without the presence of inhibitor agents, the process is carried out in general under temperature conditions of the order of 120 °C or even higher, while it is generally known that the halogen loss is favoured by higher temperatures.

Therefore, a purpose of the present invention is to provide a novel process according to which the catalytic reduction, by hydrogenation, of the nitro-halo-derivative compounds having the above defined formula (I) can be substantially carried out with nearly quantitative yields and conversions, with the substantial absence of dehalogenation phenomena.

A further purpose of the present invention is constituted by the fact that the above-said reduction is carried out by using a relatively cheap and longlife catalyst, in combination with a particular efficacious system for inhibiting the dehalogenation.

These and still further purpose which will be clearer for those skilled in the art from the following disclosure, are achieved, according to the present invention, by a process for the reduction, by means of catalytic hydrogenation, of aromatic nitro-halo-derivatives having the above defined formula (I), which process is characterized in that it is carried out by operating with supported palladium as the catalyst, and with the further presence of a dehalogenation inhibitor system costituted by at least one organic ester of phosphorous acid, and at least one hydrocarbyl-silane, which are defined in greater detail in the following.

By operating according to the process of the present invention, considerable advantages are achieved, which may be summarized as follows:

- possibility of using also palladium-based catalysts, by far less expensive and operatively more practical than those based on platinum, rhodium, and the like;
-limited dehalogenation degrees (lower than 1%), together with high conversion values (higher than 99%) of the starting compound are achieved;
-mild operating conditions as regards the values of temperature and pressure.

As above said, the reaction is carried out by operating in the presence of a catalyst of palladium, preferably supported on coal, i.e., Pd/C, a per se conventional catalyst, with a value of Pd content comprised, e.g., within the range of from 2% to 10%, of the type normally found on the market, or which can be prepared by means of known techniques.

The dehalogenation inhibitor system is constituted by:

a) at least an organic ester of phosphorous acid, having the formula (II):

$P(OR)_3$ (II)

and

b) at least one hydrocarbyl-silane having the formula (III):

$H\text{-}Si(R)_3$ (III)

in which formulae the R symbol represents a ($C_1$-$C_4$)-alkyl radical, or an aryl radical, and the R groups can be homogeneous or heterogeneous, i.e., equal to, or different from, one another, within the scope of the individual formulae (II) and (III), and in formulae (II) and (III).

As said, also mixtures of phosphitic compounds (II) associated with mixtures of silanic compounds (III) may be used. Triphenylphosphite as the compound (II) and triphenylsilane as the compound (III) are respectively preferred inside the dehalogenation inhibitor system.

Finally, also "precursors" of the silanic compounds of formula (III) can be used, i.e., compounds which, under the operating conditions of the process generate in situ the silanic compounds of formula (III). For this purpose, for example, the corresponding oligomers of silanes (III), such as di-silane, tri-silane, etc., can be used.

The compounds (II) and (III) which constitute the inhibitor system are used, according to the present invention, in a ratio by weight of phosphite (II)/silane (III) comprised within the range of from 0.1 to approximately 1, and preferably equal to approximately 0.5.

Relatively to the substrate of formula (I) to be reduced, amounts of Pd/C catalyst are used, which are comprised within the range of from 0.02% to approximately 1% by weight, and amounts of dechlorination inhibitor system (II)+(III) are used, which are comprised within the range of from 0.1 mol % to approximately 10 mol % relatively to the moles of the compound used as the starting compound (I).

Preferred amounts are approximately 0.05% by weight of (metal) Pd catalyst, and approximately 0.3 mol % for the inhibitor system (II)+(III).

The process is carried out in an organic solvent, preferably constituted by a hydrocarbon, such as benzene, cyclohexane, toluene, and so forth, of a lower alifatic ($C_1$-$C_4$)-alcohol, such as methanol, ethanol, atc.; the reaction can be carried out also in the absence of a solvent, with an excess of the starting compound (I).

The operating temperature range is comprised between 25° and approximately 100°, preferably between 50° and approximately 80° C, and the operating pressure is comprised within the range of from atmospheric pressure to approximately 25 $kg/cm^2$, preferably of from 15 to 20 $kg/cm^2$.

The concentration of the starting product (I) in the solution is not a critical parameter.

Due to the negligible evolution of hydrogen halide deriving from the dehalogenation, it is possible to operate in the absence of an inorganic base, as sodium carbonate, which is added, according to the prior art, in order to block the hydrogen halide.

The duration of the reaction of reduction may range, according to the adopted values for the reaction parameters (temperature, pressure, amount of catalyst and of inhibitor, ratios and concentration, and so forth), within wide limits.

However, useful operating times have been shown to be comprised within the range of from 0.5 to approximately 5 hours.

The process of the present invention is in particular based on the observation that the presence of silanes (III) alone, as the dechlorination inhibitor agents, led to acceptable reaction times and conversion values, but with extremely high dechlorination values, whilst the contrary occurred when phosphites (II) alone were used, together with palladium; in fact, the phosphite (II) associated with palladium may supply low dechlorination values, to the expense of a lower conversion and with longer reaction times than obtained

with silane (III).

Therefore, it results surprising that the association of the two dehalogenation inhibitor agents of formulae (II) and (III), having substantially complementary activities on the trend of the reaction of reduction of the nitro-halo-derivative having formula (I), may on the contrary perform, when are associated with each other, a synergistic action favourable to the desired trend of the reaction.

The invention is now disclosed in greater detail by the following examples supplied for the purpose of illustrating the invention without limiting it.

Examples 2 and 3 were carried out for comparative purposes, by operating in the presence of the individual phosphitic and silanic compounds, constituting, together with each other, the inhibitor system which is the subject-matter of the present invention. The synergistic effect obtained by associating the inhibitors is evident.

## Examples 1-3

To an autoclave, equipped with systems for metering and charging the reactants, and with a thermometer, 25 g of 4-chloro-3-nitro-benzotrifluoride dissolved in methyl alcohol, 0.250 g of catalyst of 5% palladium on coal, Pd/C are charged together with the amount of inhibitor, or of inhibitor system according to the present invention, planned for the relevant test (see following Table).

The autoclave is then pressurized with hydrogen at a pressure of 15-18 kg/cm$^2$, with the temperature being kept at 60°-70° C, for a time as long as shown in the table. At the end of the reaction, the aqueous phase is separated from the organic phase and this latter is washed with 20 ml of solution of $Na_2CO_3$ at 20%.

The product is then recovered according to conventional techniques.

The results obtained are shown in Table 1.

Table 1

| Example | 1 | 2 | 3 |
|---|---|---|---|
| Inhibitor System* | $P(OPh)_3$ + | $P(OPh)_3$ | - |
| | $HSi(Ph)_3$ | - | $HSi(Ph)_3$ |
| Amount (mol %) | 0.1 + | 0.5 | - |
| | 0.2 | - | 0.1 |
| Time (hours) | 3 | 10-12 | 4-6 |
| Yields % ** | | | |
| 1 | - | 36.4 | 1.3 |
| 2 | 99.04 | 62.4 | 95.3 |
| 3 | 0.56 | 0.6 | 3.3 |
| 4 | 0.04 | 0.6 | 0.1 |

* Ph = phenyl

** 1 = unreacted starting compound

2 = reduced, halogenated compound

3 = dehalogenated reduced compound

4 = other undetermined compounds

Tests 2 and 3 were repeated with the addition of 1 ml of $Na_2CO_3$ at 30%, with poorer results being obtained.

## Claims

1. Process for reducing by catalytic hydrogenation aromatic nitro-halo-derivatives having the formula (I):

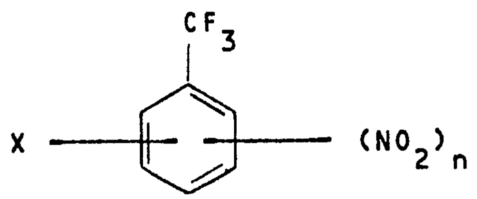

(I)

wherein X represents a halogen atom, preferably a chlorine atom, and n represents an integer selected from 1 and 2, characterized in that it is carried out in the presence of a catalyst of supported palladium, and with the further presence of a dehalogenation inhibitor system costituted by:

a) at least one organic ester of phosphorous acid, and

(b) at least one hydrocarbyl-silane.

2. Process according to claim 1, characterized in that said catalyst is constituted by palladium supported on coal.

3. Process according to claim 1, characterized in that said inhibitor system is constituted by:

a) at least an organic ester of phosphorous acid, having the formula (II):

$P(OR)_3$ (II)

and

b) at least one hydrocarbyl-silane having the formula (III):

$H\text{-}Si(R)_3$ (III)

in which formulae the R symbol represents a ($C_1$-$C_4$)-alkyl radical, or a phenyl radical, and in the individual formulae R may have meanings different from one another.

4. Process according to claim 1, characterized in that said inhibitor system is constituted by triphenyl phosphite and triphenyl silane.

5. Process according to claim 1, characterized in that said hydrocarbyl-silanic compound is prepared in situ by starting from a precursor thereof.

6. Process according to claim 3, characterized in that said inhibitor system is used according to a ratio of

phosphitic compound (II)/silane (III)

comprised within the range of from 0.1 to approximately 1.

7. Process according to claim 1, characterized in that amounts of catalyst of supported Pd are used, which are comprised within the range of from 0.02% to approximately 1% by weight.

8. Process according to claim 3, characterized in that amounts of the inhibitor system (II) + (III) are used, which are comprised within the range of from 0.1 mol % to approximately 10 mol %, relatively to the moles of the compound used as the starting compound of formula (I).

9. Process according to claim 1, characterized in that the process is carried out in an organic solvent vehicle, preferably selected from benzene, toluene, cyclohexane and the lower alifatic ($C_1$-$C_4$)-alcohols.

10. Process according to claim 1, characterized in that the process is carried out by operating at a temperature comprised within the range of from 25 °C to approximately 100 °C and under a pressure comprised within the range of from atmospheric pressure to 25 $kg/cm^2$.